(19) Europäisches Patentamt
European Patent Office
Office européen des brevets

(11) EP 2 964 603 B1

(12) **FASCICULE DE BREVET EUROPEEN**

(45) Date de publication et mention de la délivrance du brevet:
**07.04.2021 Bulletin 2021/14**

(21) Numéro de dépôt: **14713197.3**

(22) Date de dépôt: **28.02.2014**

(51) Int Cl.:
*C07C 67/03 (2006.01)* *C07C 29/74 (2006.01)*
*C07C 29/09 (2006.01)* *C11C 3/00 (2006.01)*
*C11C 3/10 (2006.01)*

(86) Numéro de dépôt international:
**PCT/FR2014/050443**

(87) Numéro de publication internationale:
**WO 2014/135769 (12.09.2014 Gazette 2014/37)**

(54) **UTILISATION D'ACIDE SULFONIQUE POUR LA RECUPERATION DE GLYCEROL ISSU DE LA REACTION DE TRANS-ESTERIFICATION DE TRIGLYCERIDES**

VERWENDUNG EINER SULFONSÄURE ZUR RÜCKGEWINNUNG VON GLYCEROL AUS EINER TRIGLYCERIDUMESTERUNGSREAKTION

USE OF SULPHONIC ACID FOR RECOVERING GLYCEROL FROM A TRIGLYCERIDE TRANSESTERIFICATION REACTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.03.2013 FR 1351995**
**07.03.2013 US 201361774252 P**
**03.12.2013 FR 1361970**

(43) Date de publication de la demande:
**13.01.2016 Bulletin 2016/02**

(73) Titulaire: **Arkema France**
**92700 Colombes (FR)**

(72) Inventeurs:
- **MONGUILLON, Bernard**
**92700 Colombes (FR)**
- **LAFFITTE, Jean-Alex**
**64200 Biarritz (FR)**

(56) Documents cités:
**EP-A1- 1 889 899** **US-A1- 2011 044 972**
**US-A1- 2012 245 371**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

EP 2 964 603 B1

## Description

La présente invention concerne l'utilisation d'acide sulfonique pour la préparation de glycérol. Plus précisément la présente invention concerne un procédé de purification de glycérol obtenu comme sous-produit de trans-estérification de triglycérides lors de la préparation d'acides gras, d'esters gras et/ou de sels d'acides gras. Cette invention permet ainsi la valorisation du glycérol issu de la trans-estérification de triglycérides. L'invention concerne également un procédé combiné de préparation d'esters d'acides gras et de glycérol à partir de glycérides utilisant au moins un acide sulfonique.

Les triglycérides sont majoritairement des triesters de glycérol et d'acides gras (ils peuvent également contenir des mono- et des di-glycérides) et sont trouvés en abondance dans la nature, par exemple dans les plantes oléagineuses et les graisses animales pour ne citer que les sources les plus importantes de triglycérides. Il existe aujourd'hui de très nombreuses industries pratiquant la trans-estérification de ces produits naturels que sont les triglycérides.

Parmi ces industries, on peut citer à titre indicatif et non limitatif les industries de fabrication de produits cosmétiques, de parfums et fragrances, de solvants organiques, de biodiesel, de savons et autres.

La réaction générale de trans-estérification des triglycérides, généralement réalisée en milieu basique, en présence d'un alcool, par exemple le méthanol, pour conduire aux esters méthyliques d'acides gras correspondants, répond au schéma suivant :

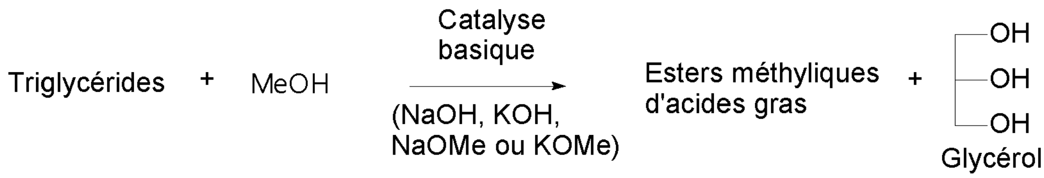


Cette réaction de trans-estérification, qui peut être réalisée relativement aisément au laboratoire, présente toutefois quelques difficultés sur le plan industriel. En effet, les triglycérides de départ contiennent la plupart du temps une quantité variable d'acides gras libres (ou « Free Fatty Acid » en langue anglaise, ou FFA).

Lorsque cette teneur en acides gras libres dans les triglycérides est relativement élevée, typiquement supérieure à environ 5% en poids, les FFA peuvent alors former, en présence du catalyseur basique, des sels d'acide gras qui agissent comme des tensio-actifs, et provoquent des mousses qui rendent difficile la séparation du glycérol des esters méthyliques d'acides gras formés.

Ce problème a été résolu, et il est maintenant connu de réaliser un prétraitement des triglycérides, qui consiste en une première estérification desdits FFA en présence de méthanol et d'un catalyseur acide, tel que l'acide sulfurique ou l'acide méthane-sulfonique (cf. FR2929621). Les FFA préalablement contenus dans les triglycérides sont alors sous forme d'esters qui ne seront pas salifiés par le catalyseur basique lors de la réaction de trans-estérification.

Les FFA présents dans les triglycérides de départ peuvent également se trouver en quantités plus faibles, typiquement entre 0,1% en poids et 5% en poids, et dans ces cas, un lavage basique peut être suffisant pour les éliminer sous forme de sels basiques.

Après la réaction de trans-estérification, les esters d'acides gras, généralement des esters méthyliques d'acides gras, sont présents dans le milieu réactionnel basique avec le glycérol. Ce milieu réactionnel peut comprendre des quantités plus ou moins importantes d'eau selon les conditions dans lesquelles la réaction de trans-estérification a été conduite. Le glycérol ainsi que l'eau éventuelle ne sont pas solubles dans les esters d'acides gras et sont séparés de ces derniers par décantation ou tout autre moyen permettant la séparation de phases.

La demande en esters d'acides gras est en constante croissance, ne serait-ce que pour la fabrication de biodiesel, et l'industrie produit aujourd'hui et plus encore demain, des quantités toujours plus importantes de ces esters. La production de glycérol est par conséquent toujours plus importante et il serait tout à fait intéressant de pouvoir mieux valoriser ce « sous-produit », dans l'objectif d'atteindre un degré de pureté très élevé, par exemple de qualité pharmaceutique.

L'art antérieur comprend ainsi de très nombreux documents concernant la préparation d'esters d'acides gras, notamment pour la production de biodiesel, par réaction de trans-estérification de triglycérides, comme par exemple décrit dans le brevet EP-B-0 658 183. Plus récemment, la demande US 2011/0245521 qui traite également de la production de biodiesel, n'évoque que très brièvement la récupération de glycérol par purification chimique et distillation pour utilisation comme matière première industrielle ou pharmaceutique. Aucune indication n'est fournie sur ladite purification chimique et la distillation du glycérol considéré comme « sous-produit ».

D'autres documents de l'art antérieur traitent déjà de la possibilité de valorisation de glycérol, comme par exemple la demande de brevet CN101423456, qui décrit un procédé de récupération et de purification de glycérol comme sous-produit de production de biodiesel. Cette purification utilise une distillation moléculaire pour obtenir un glycérol de qualité médicale. Une telle distillation de glycérol est effectuée après addition d'acide sulfurique dans le

milieu réactionnel de trans-estérification.

Comme autre exemple, la demande de brevet CN101475444 décrit également un procédé de purification de glycérol issu de la préparation de biodiesel consistant à filtrer le glycérol brut pour retirer les impuretés solides, à évaporer le glycérol filtré, puis à l'introduire dans une colonne échangeuse d'ions puis enfin le soumettre à une distillation sur couche mince. Le glycérol est obtenu avec une pureté de plus de 95%.

L'art antérieur enseigne clairement que le glycérol brut issu de la trans-estérification de triglycérides est présent dans une phase basique qui contient une quantité plus ou moins importante du surplus d'alcool utilisé pour la réaction de trans-estérification (généralement du méthanol), qui peut contenir également de l'eau, mais aussi et surtout des résidus de catalyseurs basiques tels que par exemple l'hydroxyde de sodium, l'hydroxyde de potassium, le méthylate de sodium ou le méthylate de potassium, des sels de sodium ou de potassium d'acide gras, ainsi que d'autres impuretés.

En outre, il est nécessaire d'acidifier cette phase basique, afin de pouvoir isoler un glycérol de pureté convenable. En effet, les sels de sodium et/ou de potassium d'acide gras (souvent dénommés « savons ») sont en particulier responsables de la formation de mousses plus ou moins importantes, créant ainsi une émulsion non désirée lors de la récupération de l'alcool (méthanol) par évaporation, ce qui rend difficile la conduite de cette opération sans pertes de rendement et de pureté.

Un des intérêts de cette opération d'acidification est la transformation de ces savons en acides gras libres, non tensio-actifs, et qui peuvent ensuite être séparés beaucoup plus facilement de la phase glycérol et ainsi valoriser ultérieurement ce dernier dans de bonnes conditions de pureté et de rendement.

Cette opération d'acidification est généralement effectuée par ajout d'au moins un acide fort, généralement sous forme de solution aqueuse. Les acides forts habituellement utilisés sont l'acide sulfurique, l'acide chlorhydrique et l'acide phosphorique qui génèrent respectivement des sulfates, des chlorures et des phosphates de sodium ou potassium. Chacun de ces acides présente toutefois des inconvénients, en termes de corrosion, ou de génération d'effluents néfastes pour l'environnement, pour ne citer que certains de ces inconvénients.

Qui plus est, la présence de ces sels (sulfates, chlorures et phosphates) s'avère en réalité très gênante à plusieurs titres lors de la purification du glycérol, tout particulièrement lors de sa distillation ultérieure. En effet, il a pu être observé que les sulfates, chlorures et phosphates sont peu solubles dans le glycérol et les mélanges glycérol/eau. Cette faible solubilité constatée peut nuire à la conduite de la distillation et la récupération de glycérol purifié dans des conditions de rendements et de pureté acceptables.

La présence de composés insolubles dans les flux d'une installation industrielle de distillation est hautement préjudiciable en ce que ces insolubles peuvent provoquer des perturbations des flux, notamment dans la colonne de distillation elle-même et par conséquent entraîner des pertes de charge, voire des risques de colmatage, dépôts, etc. De plus, toute perte de charge nécessite une consommation d'énergie plus importante, notamment d'opérer à une température plus élevée, ce qui a pour conséquence des dégradations et décompositions du produit, entraînant ainsi une perte de qualité du glycérol purifié et des pertes de rendement global de distillation.

La demande EP 1 889 899 A1 divulgue un procédé de production de biodiesel comprenant également une étape de récupération du glycérol, dans laquelle le glycérol brut est acidifié par un acide, de préférence un acide organique faible, tel que l'acide acétique, l'acide formique et l'acide propionique. L'utilisation d'un acide organique ne conduit pas, selon ce document, à la formation de sels insolubles. Il est indiqué que le glycérol brut doit être acidifié à un pH inférieur à 8, de préférence entre 6,5 et environ 7.

De manière similaire, la demande US 2012/0245371 propose un procédé de purification de glycérol brut alcalin obtenu comme sous-produit lors de la fabrication de biodiesel à partir de la réaction de trans-estérification d'huiles végétales et de graisses animales. Ce procédé comprend l'acidification du glycérol brut à une valeur de pH d'environ 4 à environ 6 avec un acide organique alkylcarboxylique, en présence d'eau, l'acide acétique étant le seul représentant exemplifié. Il est indiqué dans cette demande que l'acidification avec l'acide acétique n'entraîne pas la formation de solides au-dessus de la phase glycérol.

Les deux documents précédemment cités enseignent ainsi qu'un des problèmes rencontrés lors de la purification d'un brut alcalin de glycérol est la présence de sels insolubles qui se forment lors de la neutralisation ou de l'acidification du glycérol brut. Ce problème semble être résolu par l'utilisation d'un acide faible, acide acétique, formique, ou propionique et en particulier l'acide acétique, plutôt qu'un acide minéral fort, tel que acide chlorhydrique, sulfurique ou phosphorique.

Cependant l'utilisation des ces acides faibles ($pK_a$ supérieur à 3,5) ne permet pas une acidification suffisante des espèces présentes dans le glycérol brut, autrement dit ne permet pas d'atteindre une valeur de pH suffisamment faible, pour permettre une élimination efficace des savons (notamment sels alcalins d'acides gras) en les transformant en acides gras libres, et une solubilisation suffisante des autres éventuels sels et impuretés présents dans le glycérol brut.

En outre, l'art antérieur montre que l'utilisation de ces acides faibles s'accompagne de l'ajout de quantités d'eau plus ou moins importantes, nécessitant ainsi la gestion de volumes de flux importants, et le traitement ultérieur de quantités importantes d'effluents.

Le document US 2011/0044972 décrit l'utilisation de plusieurs acides, parmi lesquels l'acide méthanesulfonique,

lors de l'acidification d'un milieu contenant du glycérol. Toutefois, ce document ne traite aucunement des problèmes techniques liés à l'acidification du glycérol et reste sommaire quant à la récupération du glycérol par acidification.

Il reste donc un besoin pour améliorer le procédé de récupération du glycérol brut obtenu lors des réactions de trans-estérification des glycérides, notamment des glycérides naturels, et plus particulièrement des glycérides mis en œuvre dans la préparation de biodiesel.

Les inventeurs ont maintenant découvert que les problèmes techniques exposés ci-dessus peuvent être résolus en totalité, ou au moins en partie, grâce à la présente invention. Ainsi, un premier objectif de la présente invention consiste à fournir un procédé amélioré de récupération de glycérol purifié provenant d'un brut réactionnel issu de la trans-estérification de glycérides notamment des glycérides naturels, et plus particulièrement des glycérides mis en œuvre pour la préparation de biodiesel.

Un autre objectif consiste à fournir un procédé amélioré de récupération de glycérol provenant d'un brut réactionnel issu de la trans-estérification de glycérides, dans lequel l'opération de distillation intermédiaire du méthanol et/ou eau et la distillation finale du glycérol n'est pas perturbé pas un moussage important dans le bouilleur de distillation, et dans lequel les volumes de flux et d'effluents sont tout à fait acceptables pour une production industrielle de glycérol de haute pureté. D'autres objectifs encore apparaîtront dans l'exposé de la présente invention qui suit.

Les inventeurs ont découvert de manière tout à fait surprenante que ces objectifs peuvent être atteints, en totalité ou au moins en partie, grâce au procédé de l'invention. Ce procédé comprend une étape d'acidification par au moins un acide sulfonique qui présente l'avantage d'allier les propriétés des acides forts et des acides faibles requises pour ce procédé, c'est-à-dire permettre une acidification suffisante pour éliminer le risque de formation de mousses, et permettre la formation de sels solubles pour éliminer les risques de colmatages lors de l'opération de distillation. L'utilisation d'au moins un acide sulfonique représente un compromis tout à fait inattendu et avantageux pour la valorisation industrielle de glycérol issu de trans-estérification de triglycérides.

Il a ainsi été découvert de manière surprenante que les sulfonates, en particulier les alcane-sulfonates, et plus particulièrement les méthane-sulfonates de métaux alcalins et alcalino-terreux sont plus solubles dans le glycérol et les mélanges glycérol/eau que les autres sels de métaux alcalins et alcalino-terreux formés à partir d'autres acides forts, et en particulier les acides sulfurique, chlorhydrique et phosphorique.

Ainsi, et selon un premier aspect, la présente invention concerne l'utilisation d'au moins un acide sulfonique, de préférence au moins un acide alcane-sulfonique, pour la récupération de glycérol issu d'un brut réactionnel de trans-estérification de glycérides, notamment de triglycérides d'origine végétale et/ou animale, lors d'une étape d'acidification par ledit au moins un acide sulfonique dudit brut réactionnel à un pH strictement inférieur à 4.

Dans la présente invention, on entend par acide sulfonique les acides de formule générale $R\text{-}SO_3H$, où R représente un radical alkyle ou aryle, de préférence un radical alkyle, et dans ce dernier cas on parle d'acides alcane-sulfoniques. Les acides alcane-sulfoniques préférés pour les besoins de la présente invention sont les acides de formule $R\text{-}SO_3H$, où R représente une chaîne hydrocarbonée saturée, linéaire ou ramifiée, comportant de 1 à 4 atomes de carbone.

Les acides alcane-sulfoniques utilisables dans le cadre de la présente invention sont particulièrement choisis parmi l'acide méthane-sulfonique, l'acide éthane-sulfonique, l'acide n-propane-sulfonique, l'acide *iso*-propane-sulfonique, l'acide n-butane-sulfonique, l'acide *iso*-butane-sulfonique, l'acide sec-butane-sulfonique, l'acide *tert*-butane-sulfonique, et les mélanges de deux ou plusieurs d'entre eux en toutes proportions.

Les $pK_a$ des acides sulfoniques, en général, et alcane-sulfoniques en particulier, sont tous inférieurs à zéro, alors que les $pK_a$ des acides alkylcarboxyliques sont tous supérieurs à 3,5. Ces acides, de $pK_a$ supérieurs à 3,5, ne sont pas suffisamment forts pour assurer une acidification totale de toutes les espèces en présence dans le milieu.

Selon un mode de réalisation tout particulièrement préféré, l'acide alcane-sulfonique utilisé dans le cadre de la présente invention est l'acide méthane-sulfonique ou l'acide éthane-sulfonique, de manière tout à fait préférée l'acide utilisé est l'acide méthane-sulfonique de formule $CH_3SO_3H$.

Ainsi, l'utilisation selon la présente invention met en œuvre au moins un acide alcane-sulfonique choisi parmi les acides alcane-sulfoniques à chaîne linéaire ou ramifiée comportant de 1 à 4 atomes de carbone, et de préférence au moins de l'acide méthane-sulfonique (AMS).

Tout type de formulation comprenant au moins un acide sulfonique, de préférence alcane-sulfonique peut convenir. Il est ainsi par exemple possible d'utiliser au moins un acide sulfonique sous forme anhydre ou sous forme de solution aqueuse. En règle générale, la formulation comprend de 1% à 100% en poids d'acide(s) sulfonique(s), plus généralement de 5% à 90% en poids, en particulier de 10% à 80% en poids d'acide sulfonique, et plus particulièrement de 15% à 75% en poids, le complément à 100% étant généralement constitué d'eau. Il va sans dire que lorsque la formulation comprend 100% en poids d'acide(s) sulfonique(s), on entend que le ou les acides sulfoniques sont utilisés purs, plus précisément sont utilisés seuls, sans ajout d'autres composants de formulation.

La formulation est par exemple une formulation aqueuse qui peut être préparée sous forme de mélange concentré qui est dilué par l'utilisateur final. En variante, la formulation peut également être une formulation prête à l'emploi, c'est-à-dire qu'elle ne nécessite pas d'être diluée. On peut par exemple utiliser de l'acide méthane-sulfonique en solution

aqueuse commercialisé par la société Arkema, par exemple une solution aqueuse d'acide méthane-sulfonique à 70% en poids dans l'eau, ou encore de l'acide méthane-sulfonique anhydre ou AMSA, acronyme pour « anhydrous methane sulphonic acid » en langue anglaise.

Les inventeurs ont découvert que les sulfonates de métaux alcalins et alcalino-terreux, en particulier les méthanesulfonates de sodium et de potassium, sont plus solubles dans le glycérol ou les mélanges glycérol/eau que les sels sulfates, chlorures ou phosphates de ces mêmes cations dans ce même milieu.

Selon un mode de réalisation préférée, la présente invention concerne l'utilisation, pour la récupération de glycérol issu d'une réaction de trans-estérification de glycérides, de l'acide méthane-sulfonique (AMS) en toutes concentrations possibles, allant de l'AMSA (AMS anhydre) à des concentrations de l'ordre de 5% en poids d'AMS dans l'eau, et notamment les solutions aqueuses d'AMS à 70% en poids dans l'eau, commercialisées par la société ARKEMA.

Un autre avantage lié à l'utilisation d'un acide fort, et en particulier un acide sulfonique, de préférence alcane-sulfonique, de préférence encore méthane-sulfonique, réside dans le fait que l'ajout d'un tel acide permet de diminuer la viscosité du milieu riche en glycérol et de faciliter les mesures de pH. En effet, en présence d'eau, les acides forts, contrairement aux acides organiques dits « faibles », confèrent plus facilement un caractère hydrophile au milieu, de ce fait permettent une meilleure dissociation des sels, facilitant ainsi les mesures de pH. En outre, le fait de réduire la viscosité du milieu, la glycérine étant un produit de viscosité relativement élevée, permet d'améliorer très sensiblement la séparation de la phase aqueuse d'une part et de la phase glycérol d'autre part, par exemple par distillation, lorsque cela est nécessaire.

Par « brut réactionnel » comprenant le glycérol que l'on souhaite récupérer, on entend le milieu basique qui constitue la phase contenant le glycérol après séparation de la phase contenant les esters d'acides gras, l'ensemble des deux phases résultant de la réaction de trans-estérification de glycérides, telle que décrite par exemple dans les demandes EP 1889899 et US 2010/0186289.

Ainsi le brut réactionnel, ou milieu réactionnel brut est un mélange basique, de pH généralement compris entre 10 et 14, et qui comprend typiquement par exemple :

- du glycérol,
- le ou les alcool(s) utilisé(s) pour la réaction de trans-estérification,
- éventuellement de l'eau, ou des traces d'eau,
- le ou les catalyseur(s) basique(s) (utilisé(s) pour la réaction de trans-estérification), possiblement sous forme de traces,
- sel(s) d'acide(s) gras, possiblement sous forme de traces,
- éventuellement un ou plusieurs ester(s) d'acide(s) gras, possiblement sous forme de traces,
- éventuellement des traces de mono-, di- et/ou tri-glycérides,
- éventuellement des traces de résidus organiques hors glycérol,
- éventuellement des traces de métaux.

Par « traces », on entend des quantités généralement comprises entre quelques ppm (poids) et 5% en poids par rapport au poids total du brut réactionnel, de préférence comprises entre quelques ppm (poids) et 2% en poids par rapport au poids total du brut réactionnel, de manière tout à fait préférée comprises entre quelques ppm (poids) et 1% en poids par rapport au poids total du brut réactionnel.

Parmi les catalyseurs basiques qui sont présents, possiblement à l'état de traces dans le brut réactionnel, on peut citer tous les catalyseurs qui peuvent être utilisés pour les réactions de trans-estérifications de glycérides et en particulier les trans-estérifications de triglycérides. De préférence ces catalyseurs basiques sont choisis parmi les oxydes, hydrures, hydroxydes, carbonates, hydrogénocarbonates, acétates et autres alcoolates de métaux alcalins et alcalino-terreux, les alcoolates provenant d'alcools comportant de préférence de 1 à 5 atomes de carbone. Parmi les catalyseurs basiques, on préfère l'hydroxyde de sodium, l'hydroxyde de potassium, les alcoolates de sodium, les alcoolates de potassium. De manière tout à fait préférée les catalyseurs basiques sont choisis parmi l'hydroxyde de sodium, l'hydroxyde de potassium, le méthylate de sodium et le méthylate de potassium, ces deux derniers alcoolates étant tout particulièrement préférés.

Par « alcool utilisé pour la réaction de trans-estérification », on entend de manière générale, et à titre d'exemples indicatifs mais non limitatifs, les alcools comprenant de 1 à 10 atomes de carbone, et de préférence ceux choisis parmi méthanol, éthanol, n-propanol, iso-propanol, n-butanol, iso-butanol, 3-méthyl-1-butanol, alcool néo-pentylique, pentanol et ses isomères, hexanol et ses isomères, heptanol et ses isomères, octanol et ses isomères, nonanol et ses isomères, décanol et ses isomères, ainsi que les mélanges de deux ou plusieurs d'entre eux en toutes proportions. Parmi les alcools cités ci-dessus, préférence est donnée au méthanol et à l'éthanol, et de manière particulièrement préférée, au méthanol.

Par « sels d'acides gras », on entend les sels de métaux alcalins ou alcalino-terreux, de préférence sels de sodium et/ou sels de potassium, des acides gras issus de la réaction de trans-estérification des glycérides et qui peuvent

se retrouver à l'état de traces dans la phase glycérol après l'étape de séparation des phases glycérol d'une part et esters d'acides gras d'autre part.

De même, des traces de mono-, di- et triglycérides peuvent être présents dans la phase glycérol, notamment dans le cas où la réaction de trans-estérification a été conduite de manière partielle.

De même, un ou plusieurs esters d'acide(s) gras peuvent être présents dans le brut réactionnel basique contenant majoritairement le glycérol. Ces esters peuvent être présents dans la phase glycérol par exemple en raison de leur solubilité partielle dans cette phase, en raison d'une décantation insuffisante, d'une mauvaise séparation des deux phases, et autres.

Le brut glycérol basique peut également contenir également des traces de résidus organiques provenant des huiles de départ avant trans-estérification ou encore obtenus comme sous-produits de dégradation lors de ladite réaction de trans-estérification. Les huiles de départ soumises à trans-estérification peuvent également contenir des traces de métaux, ceux-ci pouvant être également apportés par les catalyseurs employés, l'eau ou encore l'alcool utilisé.

Enfin, de l'eau peut-être présente, à l'état de traces ou en quantités plus importantes. Des traces d'eau peuvent en effet être présentes dans les triglycérides engagés dans la réaction de trans-estérification, mais aussi et surtout l'eau peut-être apportée notamment par le catalyseur basique, par exemple lorsque celui-ci est l'hydroxyde de sodium ou de potassium sous forme de solution aqueuse.

La phase glycérol comprend ainsi, outre le glycérol d'intérêt que l'on souhaite récupérer de nombreuses autres impuretés, dont certaines peuvent être présentes à l'état solide. Selon un mode de réalisation préféré, ces impuretés solides sont séparées selon tous moyens de séparation connus de l'homme du métier, par exemple séparation physique, telle que centrifugation et/ou filtration.

Comme indiqué précédemment, cette phase glycérol est basique, en raison du ou des catalyseurs basiques mis en œuvre lors de la réaction de trans-estérification, et il convient d'acidifier cette phase basique, afin d'amener son pH à une valeur strictement inférieure à 4, de préférence inférieure à 3, par exemple à une valeur de pH voisine de 2.

À cet effet, et selon l'utilisation de la présente invention, au moins un acide sulfonique, et de préférence l'acide méthane-sulfonique, est ajouté dans ledit brut réactionnel avant de conduire une réaction de distillation visant à récupérer le glycérol contenu dans ledit brut réactionnel, qui a été éventuellement mais de préférence débarrassé comme indiqué précédemment des éléments solides présents dans celui-ci.

La quantité d'acide(s) sulfonique(s) introduite dans le brut réactionnel peut donc varier dans de larges proportions en fonction des divers éléments basiques présents dans ledit brut réactionnel. L'homme du métier saura adapter la quantité d'acide(s) sulfonique(s) à ajouter au brut réactionnel en fonction également de la concentration dudit ou desdits acides. Selon un mode de réalisation l'ajout d'acide est opéré sous agitation et le pH de la solution est contrôlé en continu. L'ajout d'acide(s) est alors stoppé lorsque le pH de la solution atteint une valeur strictement inférieure à 4, de préférence inférieure à 3, par exemple voisine de 2, valeur de pH qui permet l'acidification totale, voire quasi-totale mais suffisante, des savons et autres sels présents dans le milieu et qui pourraient conduire à un moussage lors de la distillation ultérieure.

Après cette étape d'acidification, on observe généralement l'apparition d'une phase supérieure comprenant majoritairement des acides gras libres, et des traces d'esters d'acides gras. Le milieu peut alors être soumis avantageusement et de préférence, bien que cette étape ne soit pas obligatoire, à une étape de séparation de phases (par exemple par décantation, centrifugation, aspiration, soutirage, ou toute autre méthode bien connue de l'homme du métier) afin de séparer les acides gras libres provenant des sels d'acides gras après acidification ainsi que les esters éventuellement présents.

De même, dans les cas où des sels insolubles sont présents, une étape de filtration peut être envisagée, bien que ceci ne représente pas une variante préférée du procédé de l'invention.

La solution acidifiée comprend une quantité de glycérol généralement comprise entre 30% et 99% en poids par rapport au poids total du brut réactionnel acidifié, plus fréquemment entre 50% et 95% en poids de glycérol par rapport au poids total du brut réactionnel acidifié. En outre la solution acidifiée peut contenir du méthanol, en quantités plus ou moins importantes, et de l'eau, également en quantités plus ou moins importantes.

En outre, et comme indiqué précédemment, la phase glycérol contient généralement des sels d'acide gras, plus communément dénommés « savons », en quantités plus ou moins importantes. L'acidification de cette phase glycérol permet également la transformation de ces savons en acides gras libres (FFA). Or il a été observé, et ceci représente encore un autre avantage lié à l'utilisation d'au moins un acide sulfonique, de préférence d'au moins un acide alcane-sulfonique, de préférence encore d'acide méthane-sulfonique, que l'indice d'acide (IA) de la phase glycérol après cette étape d'acidification avec au moins un acide sulfonique selon l'invention et d'élimination de ces FFA, est plus faible que lorsqu'un autre acide est utilisé.

L'avantage qui en résulte est tout à fait considérable si l'on considère que cette phase glycérol acidifiée, après élimination des FFA, doit être neutralisée à pH neutre (environ pH = 7) avant distillation. Cette neutralisation peut être effectuée selon tout moyen connu en soi, par exemple à l'aide d'une base, de préférence une base forte, avantageusement hydroxyde de sodium ou de potassium. Un indice d'acide plus faible grâce à l'acidification préalable au moyen d'au

moins un acide sulfonique conduit ainsi à une quantité moins importante de base nécessaire à la neutralisation et par conséquent à la formation d'une moindre quantité de sels.

Ainsi, cette phase acidifiée, riche en glycérol est alors neutralisée (à pH environ 7) au moyen d'au moins une base, de préférence au moins une base forte, telle que l'hydroxyde de sodium ou l'hydroxyde de potassium. Cette solution neutralisée peut alors être engagée dans l'étape de distillation du glycérol, après distillation du méthanol et de l'eau, comme indiqué plus loin.

La neutralisation de la phase acide n'est pas sans conséquences sur la nature du milieu qui est destiné à être distillé. En effet lors de cette étape de neutralisation, les espèces acides sont neutralisées sous forme de sels dans un milieu riche en glycérol.

Les inventeurs ont découvert de manière surprenante que les différents sels de métaux alcalins et/ou alcalino-terreux, présents dans cette phase ainsi neutralisée et destinée à être distillée, sont beaucoup plus solubles dans le glycérol lorsque la neutralisation a été effectuée sur un milieu préalablement acidifié à l'aide d'au moins un acide sulfonique, en particulier au moins un acide alcane-sulfonique, plus particulièrement l'acide méthane-sulfonique, alors que les mêmes sels sont beaucoup moins solubles lorsque l'acidification a été effectuée avec d'autres acides, notamment acides minéraux forts couramment utilisés dans le domaine, tels que les acides sulfurique, chlorhydrique ou phosphorique.

En outre, les sels précités sous forme de sulfonates, de préférence alcane-sulfonates, de préférence encore méthane-sulfonates, se sont montrés beaucoup plus solubles que les sulfates, chlorures et autres phosphates, non seulement dans le glycérol, mais aussi dans les mélanges glycérol/eau, quel que soit le ratio glycérol/eau et quelle que soit la température, notamment quelle que soit la température comprise dans la gamme de température de fonctionnement de la colonne de distillation.

Ceci est d'autant plus remarquable que les opérations de distillation sont très sensibles aux impuretés solides présentes dans les installations de distillation et notamment dans le bouilleur (ou pied) de distillation mais aussi dans la colonne de distillation. Or, les gradients de concentration glycérol/eau et températures varient le long de la colonne de distillation.

L'acidification par au moins un acide sulfonique, de préférence au moins un acide alcane-sulfonique, et de préférence encore par l'acide méthane-sulfonique, offre l'avantage d'une meilleure solubilité des sels, notamment des sels de sodium et/ou potassium présents dans le glycérol, et ce quel que soit le gradient glycérol/eau, ce qui signifie que la solubilité des sels est plus importante non seulement dans le bouilleur mais aussi sur toute la hauteur de la colonne.

Cet avantage de l'acide sulfonique par rapport aux autres acides forts utilisés couramment, permet ainsi d'éviter la formation de dépôts de solides qui peuvent provoquer des perturbations des flux, notamment dans la colonne de distillation elle-même et par conséquent entraîner des pertes de charge, voire des risques de colmatage, dépôts, etc.

En outre, cette plus grande solubilité des sels d'acides sulfonique dans la phase comprenant le glycérol, et notamment dans le bouilleur, en pied de colonne, permet de poursuivre l'opération de distillation jusqu'à un degré plus avancé, et ainsi améliorer encore le rendement de distillation. Un autre avantage lié à une meilleure solubilité des sels dans le glycérol est la réduction du risque de colmatage dans le bas de la colonne, là où les mélanges glycérol/eau sont les plus concentrés en glycérol. Le rendement global de la distillation se trouve ainsi grandement amélioré.

La plus grande solubilité des sels dans le milieu à distiller peut également permettre d'envisager une diminution sensible du nombre de plateaux théoriques de la colonne, et par conséquent la hauteur physique de la colonne, de même que réduire substantiellement la quantité d'énergie utilisée pour la distillation totale du glycérol.

Un autre avantage encore, lié à l'acidification par au moins un acide sulfonique du brut réactionnel contenant le glycérol, réside dans le fait que les dépôts de solides sont moins importants et par conséquent les périodes d'arrêt pour nettoyage des installations de distillation sont plus espacées dans le temps.

Un autre avantage encore est que les sulfonates, et en particulier les alcane-sulfonates et plus particulièrement les méthane-sulfonates, sont très solubles en milieu aqueux et sont biodégradables. Les installations sont par conséquent plus faciles à nettoyer, et de ce fait nécessitent des volumes d'eau beaucoup plus réduits, et les effluents de nettoyage sont plus respectueux de l'environnement.

Le glycérol présent dans la solution acidifiée, et éventuellement neutralisée, est alors séparé de l'eau et de l'alcool résiduel utilisé lors de la réaction de trans-estérification. Cette séparation peut être effectuée selon toute méthode connue de l'homme du métier et, de préférence, par distillation. Lors de cette opération de distillation, l'alcool, généralement le méthanol, est tout d'abord distillé, puis l'eau, et enfin le glycérol. La distillation du glycérol est généralement opérée sous pression réduite, notamment afin de minimiser les risques de décomposition dudit glycérol à température élevée, par exemple environ 10 mbar (environ 1 kPa), le point d'ébullition du glycérol étant alors d'environ 160°C, ou bien entre 15 mbars et 150 mbars (entre 1,5 kPa et 15 kPa), le point d'ébullition du glycérol étant alors compris entre environ 160°C et environ 280°C.

Selon un autre aspect, la présente invention concerne un procédé amélioré de récupération de glycérol à partir d'un brut réactionnel issu de la réaction de trans-estérification de triglycérides, comprenant au moins les étapes suivantes :

a) fourniture d'un brut réactionnel basique riche en glycérol et contenant également de l'eau et un alcool, par exemple du méthanol ;
b) éventuelle séparation des impuretés solides ;
c) acidification du brut réactionnel issu de l'étape a), avec au moins un acide sulfonique jusqu'à une valeur de pH inférieure ou égale à 4, de préférence inférieure ou égale à 3, par exemple voisine de 2 ;
d) éventuelle décantation et séparation de la phase riche en glycérol et de la phase riche en acides gras libres et en esters d'acides gras ;
e) neutralisation jusqu'à pH d'environ 7, à l'aide d'une base, par exemple une base minérale forte, de préférence choisie parmi l'hydroxyde de sodium et l'hydroxyde de potassium ;
f) distillation de l'alcool et de l'eau ;
g) distillation du glycérol, de préférence sous pression réduite ;
h) récupération du glycérol distillé.

Selon une variante du procédé amélioré de récupération du glycérol, l'étape f) de distillation du méthanol et de l'eau peut être effectuée avant l'étape e) de neutralisation. Il est également possible de réaliser la distillation du méthanol, puis opérer l'étape de neutralisation, et conduire l'étape de distillation de l'eau puis du glycérol.

Selon encore un autre aspect, la présente invention concerne un procédé combiné de préparation d'esters d'acides gras ou de mélanges d'esters d'acides gras d'une part et de glycérol d'autre part, comprenant au moins les étapes suivantes :

1) fourniture de triglycérides d'origine végétale et/ou animale,
2) trans-estérification, en milieu basique, desdits triglycérides, en présence d'au moins un alcool comprenant de 1 à 10 atomes de carbone, de préférence de 1 à 5 atomes de carbone, de préférence en présence de méthanol, pour obtenir d'une part une phase riche en esters d'acides gras ou mélanges d'esters d'acides gras et d'autre part une phase riche en glycérol,
3) séparation des phases riche en esters et riche en glycérol, par exemple par décantation,

4a) récupération des esters et mélanges d'esters d'acides gras, après acidification, avec au moins un acide sulfonique, de la phase comprenant lesdits esters et mélanges d'esters d'acide gras, et
4b) récupération du glycérol selon le procédé décrit plus haut, étape a) à étape h), comprenant l'acidification de la phase comprenant le glycérol avec au moins un acide sulfonique.

Ce procédé permet la production combinée à la fois de glycérol de haute pureté d'une part et d'esters d'acides gras d'autre part, à partir d'huiles, en particulier d'huiles végétales ou animales qui contiennent de grandes quantités de triglycérides.

Ce procédé est caractérisé par le fait qu'il met en œuvre au moins un acide sulfonique, de préférence au moins un acide alcane-sulfonique et de préférence encore l'acide méthane-sulfonique, à la fois pour l'acidification de la phase riche en esters d'acides gras et pour l'acidification de la phase riche en glycérol. L'utilisation du même acide pour l'acidification des deux phases précitées, présente, entre autres, l'avantage d'une plus grande simplicité de mise en œuvre de l'opération industrielle, l'avantage de disposer d'un acide non corrosif et biodégradable, respectueux de l'environnement, et l'avantage de solubiliser les sels présents dans la phase riche en glycérol permettant la conduite de la distillation finale de glycérol dans des conditions plus économiques, comme cela a été expliqué plus haut dans la description.

Le glycérol distillé de haute pureté ainsi obtenu peut par exemple être utilisé tel quel comme solvant, comme composant de formulations sanitaires ou de nettoyage, ou encore comme précurseur ou intermédiaire de synthèse dans de nombreux domaines d'applications, tels que par exemple la préparation de produits pharmaceutiques et vétérinaires, de produits cosmétiques, de produits phytosanitaires et autres.

Les esters et mélanges d'esters d'acides gras peuvent trouver quant à eux de nombreuses applications, et par exemple peuvent être utilisés comme solvants, tensio-actifs, précurseurs de monomères, précurseurs d'alcool gras, lubrifiants ou encore comme constituants de biodiesel, c'est-à-dire pour la fabrication de carburant de type diesel comprenant une quantité plus ou moins importante de composés d'origine renouvelable, tel que le carburant diesel répondant à la norme européenne.

Les exemples suivants illustrent la présente invention sans toutefois en limiter sa portée définie par les revendications qui suivent.

**Exemple 1** : Solubilités des sels de sodium dans le glycérol

Dans un tricol de 50 mL équipé d'un agitateur magnétique, d'un réfrigérant et d'une sonde de température,

sont introduits 30 g de mélange glycérol/eau. Le sel à tester est ensuite ajouté par portions de 0,1 g environ jusqu'à apparition de cristaux non dissous (saturation du milieu) Le pourcentage de sel dissous est calculé suivant la formule suivante :

$$\% \text{ sel dissous} = m / (M + m)$$

où M représente la masse de mélange glycérol/eau, et m représente la masse de sel introduite.

Les sels testés pour leur solubilité dans les mélanges glycérol/eau sont les sels de sodium des acides sulfurique ($pK_a$ = -9), chlorhydrique ($pK_a$ = -6,3), acétique ($pK_a$ = +4,75) et méthane-sulfonique ($pK_a$ = -1,9).

Il a ainsi pu être observé que la quantité maximale, à 20°C, et 100°C respectivement, dans un mélange glycérol/eau 90/10 en poids, de sel solubilisé est de :

- 2,5 % (à 20°C et à 100°C) dans le cas du sel formé avec l'acide sulfurique (sulfate de sodium) ;
- 8 % et 9,4 % dans le cas du sel formé avec l'acide chlorhydrique (chlorure de sodium) ;
- 15 % et 30 % dans le cas du sel formé avec l'acide acétique (acétate de sodium) ;
- 15 % et 22 % dans le cas du sel formé avec l'acide méthane-sulfonique (méthane-sulfonate de sodium).

La solubilité maximale du méthane-sulfonate de sodium atteint la valeur de 22 % dans un mélange glycérol/eau 90/10 en poids, à 100°C.

Les résultats ci-dessus montrent que les sels de sodium de l'acide méthane-sulfonique sont 2 à 10 fois plus solubles dans les mélanges glycérol/eau que respectivement le sulfate de sodium et le chlorure de sodium. On observe en outre une solubilité du même ordre de grandeur pour les sels d'acide méthane-sulfonique et les sels d'acide acétique, un acide organique faible.

On réalise un test similaire de solubilité dans le glycérol, cette fois-ci à 180°C (sans eau). À cette température de 180°C, on observe que la quantité maximale dans le glycérol de sel solubilisé est de :

- 0,6 % dans le cas du sel de sodium formé avec l'acide sulfurique ;
- 7,5 % dans le cas du sel de sodium formé avec l'acide chlorhydrique ; et
- 26 % dans le cas du sel formé de sodium avec l'acide méthane-sulfonique.

**Exemple 2** : Étude comparative de divers acides sur une phase glycérol brute

Pour cette étude, on utilise une phase brute de glycérol obtenue après transestérification à l'hydroxyde de sodium d'une huile de colza et séparation de la phase contenant les esters méthyliques (biodiesel).

On mélange une quantité de 40 g de cette phase brute de glycérol avec 4 g d'eau, à température ambiante. Chacun des acides à tester est ajouté en 15 minutes, jusqu'à obtenir une valeur de pH d'environ 4. Le milieu réactionnel est agité mécaniquement puis laissé à décanter pendant une période d'environ 4 heures. La phase supérieure contient principalement des acides gras libres (FFA) et des traces d'esters d'acides gras, tandis que la phase inférieure contient principalement du glycérol (phase riche en glycérol).

Les acides suivants sont testés comparativement :

- acide méthane sulfonique (AMS) à 70% en poids dans l'eau (Arkema) ;
- acide phosphorique ($H_3PO_4$) à 75% en poids dans l'eau ;
- acide sulfurique ($H_2SO_4$) à 95% en poids dans l'eau ;
- acide citrique (ACi) à 48% en poids dans l'eau ;
- acide acétique (AAc) pur ;
- acide chlorhydrique (HCl) à 37% en poids dans l'eau.

Pour chaque test sont mesurés :

- la quantité d'acide ajoutée en % en poids par rapport au poids de la phase glycérol brute (40 g) ;
- l'augmentation de la température du milieu réactionnel (exothermie, $\Delta T$ en °C) ;
- la quantité d'insolubles en % en poids par rapport au poids de la phase glycérol brute (40 g) ;
- la quantité de phase glycérol récupérée en % en poids par rapport au poids total des deux phases présentes ; et
- l'indice d'acide de la phase riche en glycérol.

L'indice d'acide (IA) est mesuré par neutralisation de l'acidité du milieu par une base forte (hydroxyde de

potassium ou de sodium par exemple). L'indice d'acide est défini en mg de KOH consommé par gramme d'échantillon.

La méthode mise en œuvre est la suivante : dans un bêcher équipé d'un agitateur et d'une une électrode de mesure de pH (DG111 de Mettler pour milieu aqueux), est introduit environ exactement 1 g d'échantillon de phase riche en glycérol (masse m) auquel on ajoute environ 50 mL d'eau déminéralisée. Une solution aqueuse d'hydroxyde de sodium 0,1 mol/L est alors ajoutée goutte à goutte, sous agitation, jusqu'à pH 12. L'équivalence est indiquée par un saut de pH qui donne le volume équivalent v exprimé en mL. L'indice d'acide (IA), exprimé en mg de KOH, est calculé par la formule suivante :

$$IA = \frac{v \times 0,1 \times 56}{m}$$

avec v en mL, et m en grammes.

Les résultats sont présentés dans le tableau 1 suivant :

**-- Tableau 1 --**

| ***acide*** | ***AMS*** | ***$H_3PO_4$*** | ***$H_2SO_4$*** | ***AcI*** | ***AAc*** | ***HCl*** |
|---|---|---|---|---|---|---|
| ***quantité ajoutée (% poids)*** | 11,5 | 11,3 | 4,5 | 19,5 | 15,0 | 9,3 |
| ***$\Delta$T (°C)*** | 6 | 6 | 12 | 8 | 2 | 8 |
| ***Insolubles (% poids)*** | 0 | 3,4 | 0 | 0 | 0 | 0 |
| ***IA (en mg de KOH/g)*** | 1 | 61 | 2 | 37 | 92 | 2 |
| ***phase glycérol (% poids)*** | 68 | 63 | 32 | 65 | 64 | 67 |

La quantité d'acide ajoutée est sensiblement identique pour l'AMS, l'acide phosphorique et l'acide chlorhydrique. Cette quantité est environ deux fois plus élevée pour les acides organiques faibles (citrique et acétique), et deux fois plus faible pour l'acide sulfurique. Cependant, avec l'acide sulfurique, on observe la formation d'un gel qui est nuisible à une bonne séparation des phases, et entraîne une récupération d'une quantité deux fois moindre de glycérol.

En outre, la forte exothermie constatée dans le test avec l'acide sulfurique peut s'avérer gênante sur le plan industriel, nécessitant des précautions lors de l'acidification, y compris des installations particulières, par exemple module de refroidissement.

La quantité d'insolubles produite dans le test avec l'acide phosphorique rend cet acide peu convenable, les insolubles étant tout particulièrement gênants, notamment lors de l'étape ultérieure de purification par distillation du glycérol, comme indiqué précédemment.

Ces résultats montrent également que l'indice d'acide (IA) de la phase glycérol acidifiée est plus faible dans le cas de l'AMS, de l'$H_2SO_4$ et de l'HCl. Ceci représente un avantage particulièrement intéressant, en vue de la neutralisation ultérieure de cette phase glycérol par une base forte jusqu'à pH 7, avant distillation.

L'utilisation d'acide chlorhydrique n'est pas souhaitée sur le plan industriel en raison des problèmes de corrosion liés à cet acide. En outre, à 37% dans l'eau qui représente la concentration maximale possible, l'acide chlorhydrique apporte une quantité d'eau très importante, ce qui s'avère peu économique lors de la distillation ultérieure du glycérol.

Ces résultats montrent le très grand avantage lié à l'utilisation d'AMS, par rapport à d'autres acides, dans les procédés industriels de production de glycérol à partir de triglycérides, mais aussi dans les procédés industriels de production combinée de biodiesel et de glycérol à partir de triglycérides :

- l'AMS est un acide respectueux de l'environnement, et peu corrosif ;
- les sels formés avec cet acide sont beaucoup plus solubles dans le glycérol ;
- la quantité nécessaire d'AMS pour acidifier la phase brute de glycérol et l'exothermie due à l'acidification sont tout à fait comparables avec celles observées avec l'acide phosphorique, sans pour autant générer d'insolubles ;
- l'indice d'acide de la phase riche en glycérol est très faible, ce qui conduit à une quantité de base plus faible pour la neutralisation ultérieure et en conséquence une moindre quantité de sels de neutralisation ; et enfin
- une quantité récupérée de phase riche en glycérol tout à fait importante.

L'AMS représente ainsi une alternative tout particulièrement avantageuse, notamment aux acides phosphorique, sulfurique ou chlorhydrique pour la production de glycérol à partir de triglycérides.

## Revendications

1. Utilisation d'au moins un acide sulfonique, de préférence au moins un acide alcane-sulfonique, pour la récupération de glycérol issu d'un brut réactionnel de trans-estérification de glycérides, notamment de triglycérides d'origine végétale et/ou animale, lors d'une étape d'acidification par ledit au moins un acide sulfonique dudit brut réactionnel à un pH strictement inférieur à 4.

2. Utilisation selon la revendication 1, dans laquelle ledit au moins un acide sulfonique répond à la formule générale $R-SO_3H$, où R représente un radical alkyle ou aryle, de préférence un radical alkyle.

3. Utilisation selon l'une quelconque des revendications précédentes, dans laquelle ledit au moins un acide sulfonique répond à la formule générale $R-SO_3H$, où R représente une chaîne hydrocarbonée saturée, linéaire ou ramifiée, comportant de 1 à 4 atomes de carbone.

4. Utilisation selon l'une quelconque des revendications précédentes, dans laquelle ledit au moins un acide sulfonique est l'acide méthane-sulfonique ($CH_3SO_3H$).

5. Utilisation selon l'une quelconque des revendications précédentes, dans laquelle ledit au moins un acide sulfonique est sous forme anhydre ou sous forme de solution aqueuse comprenant de 5% à 90% en poids, en particulier de 10% à 80% en poids d'acide sulfonique, et plus particulièrement de 15% à 75% en poids, le complément à 100% étant constitué d'eau.

6. Utilisation selon l'une quelconque des revendications précédentes dans laquelle ledit au moins un acide sulfonique est utilisé pour l'acidification d'un brut réactionnel de trans-estérification de glycérides comprenant :

   - du glycérol,
   - le ou les alcool(s) utilisé(s) pour la réaction de trans-estérification,
   - éventuellement de l'eau, ou des traces d'eau,
   - le ou les catalyseur(s) basique(s) (utilisé(s) pour la réaction de trans-estérification), possiblement sous forme de traces,
   - sel(s) d'acide(s) gras, possiblement sous forme de traces,
   - éventuellement un ou plusieurs ester(s) d'acide(s) gras, possiblement sous forme de traces,
   - éventuellement des traces de mono-, di- et/ou tri-glycérides,
   - éventuellement des traces de résidus organiques hors glycérol,
   - éventuellement des traces de métaux.

7. Procédé de récupération de glycérol à partir d'un brut réactionnel issu de la réaction de trans-estérification de triglycérides, comprenant au moins les étapes suivantes :

   a) fourniture d'un brut réactionnel basique riche en glycérol et contenant également de l'eau et un alcool, par exemple du méthanol ;
   b) éventuelle séparation des impuretés solides ;
   c) acidification du brut réactionnel issu de l'étape a), avec au moins un acide sulfonique jusqu'à une valeur de pH inférieure à 4, de préférence inférieure à 3, par exemple voisine de 2 ;
   d) éventuelle décantation et séparation de la phase riche en glycérol et de la phase riche en acides gras libres et en esters d'acides gras ;
   e) neutralisation jusqu'à pH d'environ 7, à l'aide d'une base, par exemple une base minérale forte, de préférence choisie parmi l'hydroxyde de sodium et l'hydroxyde de potassium ;
   f) distillation de l'alcool et de l'eau ;
   g) distillation du glycérol, de préférence sous pression réduite ;
   h) récupération du glycérol distillé, l'étape f) pouvant être réalisée avant l'étape e).

8. Procédé selon la revendication 7, dans lequel le au moins un acide sulfonique est l'acide méthane-sulfonique.

9. Procédé combiné de préparation d'esters d'acides gras ou de mélanges d'esters d'acides gras d'une part et de glycérol d'autre part, comprenant au moins les étapes suivantes :

   1) fourniture de triglycérides d'origine végétale et/ou animale,

2) trans-estérification, en milieu basique, desdits triglycérides, en présence d'au moins un alcool comprenant de 1 à 10 atomes de carbone, de préférence en présence de méthanol, pour obtenir d'une part une phase riche en esters d'acides gras ou mélanges d'esters d'acides gras et d'autre part une phase riche en glycérol,
3) séparation des phases riche en esters d'une part et riche en glycérol d'autres, par exemple par décantation,

4a) récupération des esters et mélanges d'esters d'acides gras, après acidification, avec au moins un acide sulfonique, de la phase comprenant lesdits esters et mélanges d'esters d'acide gras, et
4b) récupération du glycérol selon le procédé de la revendication 7, comprenant l'acidification de la phase comprenant le glycérol avec au moins un acide sulfonique.

**10.** Procédé selon la revendication 9, dans lequel ledit au moins un acide sulfonique de l'étape 4b est l'acide méthane-sulfonique.

**11.** Procédé selon la revendication 9 ou la revendication 10, dans lequel ledit au moins un acide sulfonique de l'étape 4a est l'acide méthane-sulfonique.

**Patentansprüche**

**1.** Verwendung mindestens einer Sulfonsäure, vorzugsweise mindestens einer Alkansulfonsäure, zur Rückgewinnung von Glycerin aus einem Reaktionsrohprodukt der Umesterung von Glyceriden, insbesondere von Glyceriden pflanzlicher und/oder tierischer Herkunft, bei einem Schritt des Ansäuerns des Reaktionrohprodukts mit der mindestens einen Sulfonsäure auf einen pH-Wert, der streng unter 4 liegt.

**2.** Verwendung nach Anspruch 1, wobei die mindestens eine Sulfonsäure der allgemeinen Formel $R-SO_3H$ entspricht, wobei R für einen Alkyl- oder Arylrest, vorzugsweise einen Alkylrest, steht.

**3.** Verwendung nach einem der vorhergehenden Ansprüche, wobei die mindestens eine Sulfonsäure der allgemeinen Formel $R-SO_3H$ entspricht, wobei R für eine gesättigte, lineare oder verzweigte Kohlenwasserstoffkette mit 1 bis 4 Kohlenstoffatomen steht.

**4.** Verwendung nach einem der vorhergehenden Ansprüche, wobei es sich bei der mindestens einen Sulfonsäure um Methansulfonsäure ($CH_3SO_3H$) handelt.

**5.** Verwendung nach einem der vorhergehenden Ansprüche, wobei die mindestens eine Sulfonsäure in wasserfreier Form oder in Form einer wässrigen Lösung, die 5 bis 90 Gew.-%, insbesondere 10 bis 80 Gew.-%, Sulfonsäure und spezieller 15 bis 75 Gew.-% umfasst, wobei der Rest auf 100 % aus Wasser besteht.

**6.** Verwendung nach einem der vorhergehenden Ansprüche, wobei die mindestens eine Sulfonsäure zum Ansäuern eines Reaktionsrohprodukts der Umesterung von Glyceriden verwendet wird, das Folgendes umfasst:

- Glycerin,
- den Alkohol bzw. die Alkohole, der bzw. die für die Umesterungsreaktion verwendet wird bzw. werden,
- gegebenenfalls Wasser oder Spuren von Wasser,
- den basischen Katalysator bzw. die basischen Katalysatoren (der bzw. die für die Umesterungsreaktion verwendet wird bzw. werden), möglicherweise in Form von Spuren,
- Fettsäuresalz bzw. Fettsäuresalze, möglicherweise in Form von Spuren,
- gegebenenfalls einen oder mehrere Fettsäureester, möglicherweise in Form von Spuren,
- gegebenenfalls Spuren von Mono-, Di- und/oder Triglyceriden,
- gegebenenfalls Spuren von organischen Rückständen außer Glycerin,
- gegebenenfalls Spuren von Metallen.

**7.** Verfahren zur Rückgewinnung von Glycerin aus einem Reaktionsrohprodukt der Umesterung von Triglyceriden, das mindestens die folgenden Schritte umfasst:

a) Bereitstellen eines basischen Reaktionsrohprodukts, das reich an Glycerin ist und außerdem besser und einen Alkohol, beispielsweise Methanol, enthält;
b) gegebenenfalls Abtrennen der festen Verunreinigungen;

c) Ansäuern des Reaktionsrohprodukts aus Schritt a) mit mindestens einer Sulfonsäure bis zu einem pH-Wert von weniger als 4, vorzugsweise weniger als 3, beispielsweise in der Nähe von 2;
d) gegebenenfalls Absetzenlassen und Trennen der Phase, die reich an Glycerin ist, und der Phase, die reich an freien Fettsäuren und an Fettsäureestern ist;
e) Neutralisieren bis zu einem pH-Wert von ungefähr 7 mit einer Base, beispielsweise einer starken anorganischen Base, die vorzugsweise aus Natriumhydroxid und Kaliumhydroxid ausgewählt wird;
f) Abdestillieren des Alkohols und des Wassers;
g) Abdestillieren des Glycerins, vorzugsweise unter vermindertem Druck;
h) Gewinnen des destillierten Glycerins,

wobei Schritt f) vor Schritt e) durchgeführt werden kann.

**8.** Verfahren nach Anspruch 7, wobei es sich bei der mindestens einen Sulfonsäure um Methansulfonsäure handelt.

**9.** kombiniertes Verfahren zur Herstellung von Fettsäureestern oder Gemischen von Fettsäureestern einerseits und Glycerin andererseits, das mindestens die folgenden Schritte umfasst:

1) Bereitstellen von Triglyceriden pflanzlicher und/oder tierischer Herkunft,
2) Umestern der Triglyceride in basischem Medium in Gegenwart von mindestens einem Alkohol mit 1 bis 10 Kohlenstoffatomen, vorzugsweise in Gegenwart von Methanol, unter Erhalt einer Phase, die reich an Fettsäureestern oder Gemischen von Fettsäureestern ist, einerseits und einer Phase, die reich an Glycerin ist, andererseits,
3) Trennen der Phasen, die einerseits reich an Estern sind und andererseits reich an Glycerin sind, beispielsweise durch Absetzenlassen,

4a) Gewinnen der Fettsäureester und Gemische von Fettsäureestern nach Ansäuern der Phase, die die Fettsäureester und Gemische von Fettsäureestern umfasst, mit mindestens einer Sulfonsäure und
4b) Gewinnen des Glycerins gemäß dem Verfahren nach Anspruch 7, das das Ansäuern der Phase, die das Glycerin umfasst, mit mindestens einer Sulfonsäure umfasst.

**10.** Verfahren nach Anspruch 9, wobei es sich bei der mindestens einen Sulfonsäure von Schritt 4b um Methansulfonsäure handelt.

**11.** Verfahren nach Anspruch 9 oder Anspruch 10, wobei es sich bei der mindestens einen Sulfonsäure von Schritt 4a um Methansulfonsäure handelt.

## Claims

**1.** Use of at least one sulfonic acid, preferably at least one alkane sulfonic acid, for recovering glycerol resulting from a reaction crude from transesterification of glycerides, in particular of triglycerides of vegetable and/or animal origin, during a step of acidification by said at least one sulfonic acid of said reaction crude at a pH of strictly below 4.

**2.** Use according to Claim 1, in which said at least one sulfonic acid corresponds to the general formula $R-SO_3H$, where R represents an alkyl or aryl radical, preferably an alkyl radical.

**3.** Use according to either one of the preceding claims, in which said at least one sulfonic acid corresponds to the general formula $R-SO_3H$, where R represents a linear or branched, saturated hydrocarbon-based chain containing from 1 to 4 carbon atoms.

**4.** Use according to any one of the preceding claims, in which said at least one sulfonic acid is methanesulfonic acid ($CH_3SO_3H$).

**5.** Use according to any one of the preceding claims, in which said at least one sulfonic acid is in anhydrous form or in the form of an aqueous solution comprising from 5% to 90% by weight, in particular from 10% to 80% by weight of sulfonic acid, and more particularly from 15% to 75% by weight, the rest to 100% consisting of water.

**6.** Use according to any one of the preceding claims, in which said at least one sulfonic acid is used for the acidification

of a reaction crude from transesterification of glycerides, comprising:

- glycerol,
- the alcohol(s) used for the transesterification reaction,
- optionally water, or traces of water,
- the basic catalyst(s) (used for the transesterification reaction), possibly in trace form,
- fatty acid salt(s), possibly in trace form,
- optionally one or more fatty acid ester(s), possibly in trace form,
- optionally traces of mono-, di- and/or triglycerides,
- optionally traces of organic residues other than glycerol,
- optionally traces of metals.

**7.** Process for recovering glycerol from a reaction crude resulting from the triglyceride transesterification reaction, comprising at least the following steps:

a) provision of a basic reaction crude which is rich in glycerol and also contains water and an alcohol, for example methanol;
b) optional separation of the solid impurities;
c) acidification of the reaction crude resulting from step a), with at least one sulfonic acid, to a pH value less than 4, preferably less than 3, for example close to 2;
d) optional decanting and separation of the phase rich in glycerol and of the phase rich in free fatty acids and in fatty acid esters;
e) neutralization to a pH of approximately 7, using a base, for example a strong inorganic base, preferably chosen from sodium hydroxide and potassium hydroxide;
f) distillation of the alcohol and of the water;
g) distillation of the glycerol, preferably under reduced pressure;
h) recovery of the glycerol distilled,

it being possible for step f) to be carried out before step e) .

**8.** Process according to Claim 7, in which the at least one sulfonic acid is methanesulfonic acid.

**9.** Combined process for preparing, on the one hand, fatty acid esters or mixtures of fatty acid esters and, on the other hand, glycerol, comprising at least the following steps:

1) provision of triglycerides of vegetable and/or animal origin,
2) transesterification, in a basic medium, of said triglycerides, in the presence of at least one alcohol comprising from 1 to 10 carbon atoms, preferably in the presence of methanol, so as to obtain, on the one hand, a phase rich in fatty acid esters or mixtures of fatty acid esters and, on the other hand, a phase rich in glycerol,
3) separation of the phases which are, on the one hand, rich in esters and, on the other hand, rich in glycerol, for example by decanting,

4a) recovery of the fatty acid esters and mixtures of fatty acid esters, after acidification, with at least one sulfonic acid, of the phase comprising said fatty acid esters and mixtures of fatty acid esters, and
4b) recovery of the glycerol according to the process of Claim 7, comprising the acidification of the phase comprising the glycerol with at least one sulfonic acid.

**10.** Process according to Claim 9, in which said at least one sulfonic acid of step 4b is methanesulfonic acid.

**11.** Process according to Claim 9 or Claim 10, in which said at least one sulfonic acid of step 4a is methanesulfonic acid.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2929621
- EP 0658183 B
- US 20110245521 A
- CN 101423456
- CN 101475444
- EP 1889899 A1
- US 20120245371 A
- US 20110044972 A
- EP 1889899 A
- US 20100186289 A